# EUROPEAN PATENT APPLICATION

(11) **EP 4 332 575 A1**
(43) Date of publication of application: **06.03.2024**
(21) Application number: 22774386.1
(22) Date of filing: 15.03.2022
(51) Int. Cl.: G01N 33/53, G01N 33/68, G01N 33/577

(54) **METHODS FOR DIAGNOSIS AND PROGNOSIS OF ALZHEIMER'S DISEASE**

(30) Priority: 25.03.2021 ES 202130265
(71) Applicant: Universidad de Castilla La Mancha, 02071 Albacete (ES)
(72) Inventor: PEDRERO PRIETO, Cristina María, 13071 Ciudad Real (ES); PEINADO MENA, Juan Ramón, 13071 Ciudad Real (ES); RABANAL RUIZ, Yoana, 13071 Ciudad Real (ES)
(74) Representative: Pons
(86) International application number: PCT/ES2022/070143
(87) International publication number: WO 2022/200654

(57) **Abstract**

The present invention relates to biomarkers and methods for use thereof in the diagnosis and/or prognosis of Alzheimer's disease (AD). More specifically, it relates to the use of a panel of biomarkers in an in vitro method in which the expression levels of said panel of biomarkers in a biological sample from a subject are indicative of the subject's risk of developing the disease.

## Description

### FIELD OF THE INVENTION

The present invention relates to biomarkers and methods for use in the diagnosis and/or prognosis of Alzheimer's disease (AD). More specifically, it relates to the use of a panel of biomarkers in an in vitro method in which the expression levels of said panel of biomarkers in a biological sample from a subject are indicative of the subject's risk of developing the disease.

### BACKGROUND OF THE INVENTION

AD is probably the most studied neurodegenerative disease due to its high prevalence among the world population. Various studies have characterized the pathology of AD from a cognitive perspective and/or using neuroimaging approaches. Although these approaches are necessary to distinguish the various stages of AD progression, there is still a need to identify reliable biomarkers for the effective diagnosis and prognosis of AD.

Most studies intended for the detection of AD biomarkers have been conducted in biological fluids such as blood or cerebrospinal fluid (CSF). The use of CSF represents the best approach for identifying AD biomarkers since this fluid directly contacts the interstitial fluid of the brain and accurately reflects biochemical changes related to processes of the central nervous system (CNS). In fact, CSF studies in AD have consistently demonstrated that the β-amyloid peptide (βA42), total Tau protein (T-tau), and phosphorylated Tau protein (P-tau) constitute CSF biomarkers that are relevant for the diagnosis of AD.

However, there is a clear need to identify reliable biomarkers associated with the different stages of AD, from asymptomatic stages to advanced AD, and to subsequently design optimized methods for the diagnosis and prognosis of the disease.

### DETAILED DESCRIPTION OF THE INVENTION

A complex bioinformatic and statistical analysis of the biomarkers described in the state of the art has led the authors to the discovery and characterization of a panel of 27 biomarkers, the expression of which differs in a clear and consistent manner in AD (Table I).

**Table I. Panel of protein markers.**

| **Group** | **Uniprot protein name** | **Uniprot reference** | **Documented findings** | **References** |
|---|---|---|---|---|
| Proteins that decrease with the severity of AD | Beta-amyloid 1-40 | | ↓ AD | (1) |
| | Beta-amyloid 1-42 | | | (1) |
| | APOA1 | P02647 | | (2-8) |
| | APOC2 | P02655 | | (3, 9, 10) |
| | MUC18 | P43121 | | (3, 11-13) |
| | ALBU | P02768 | | (3, 4, 7, 14) |
| | RET4 | P02753 | | (3, 7, 15, 16) |
| | N PTX2 | P47972 | | (9, 12) |
| | VGF | O15240 | | (2, 9, 10, 14, 17-26) |
| | PCSK1N | Q9UHG2 | | (3, 12, 14, 16, 27, 28) |
| | CERU | P00450 | | (3, 10, 29, 30) |
| | ANGT | P01019 | | (28, 31) |
| Proteins that increase with the severity of AD | pTau (Thr181) | | ↑ AD | (1) |
| | Tau (total) | P 10636 | | (1) |
| | APOE | P02649 | | (1) |
| | CLUS | P10909 | | (4, 10, 16, 19, 22, 32, 33) |
| | SPARC | P09486 | | (2, 3, 34) |
| | YKL40/CH3L1 | P36222 | | (2, 10, 32, 35-37) |
| | MDHC | P40925 | | (32, 36, 38) |
| | PEDF | P36955 | | (4, 6, 31) |
| | SPON1 | Q9HCB6 | | (13, 19, 28) |
| | CO3 | P01024 | | (16, 23-25, 37, 39) |
| | OSTP | P10451 | | (13, 19, 29, 30, 32, 36) |
| | ALDOA | P04075 | | (13, 29, 30, 38, 40) |
| | SMOC1 | Q9H4F8 | | (13, 29, 30, 38, 41) |
| | A2MG | P01023 | | (10, 13, 39, 42) |
| | A1AT | P01009 | | (4, 7, 19, 39) |

The biomarkers in the panel have been identified through various bioinformatic analyses of independent proteomic studies and data compiled from CSF samples of 877 subjects diagnosed with AD (average age of 70) and 1254 control subjects of advanced age without dementia or AD (average age of 74).

The initial characteristics for defining healthy control subjects according to cognitive criteria were: CDR = 0, without neuropsychological deficits and/or without meeting the criteria of mild cognitive deterioration (MCD); score ≥ 23 in the Montreal Cognitive Assessment; MMSE score of 30; and asymptomatic NC, not carriers of mutations.

The reference criteria for defining AD were the presence of cognitive failures reported by the subject; the cognitive criteria were CDR ≥ 1; score < 23 in the Montreal Cognitive Assessment, and subjects who fulfilled the criteria of probable AD dementia according to the NIA-AA criteria. Furthermore, most publications confirmed the diagnosis of AD using the CSF biomarkers, βA42, T-tau and/or P-tau, and provided clinical evaluations including the following: informant-based detailed history, assessment of medical records, medical history, family history, physical and neurological examination, routine laboratory tests, CT or MRI of the brain, neurological and cognitive brain imaging examinations.

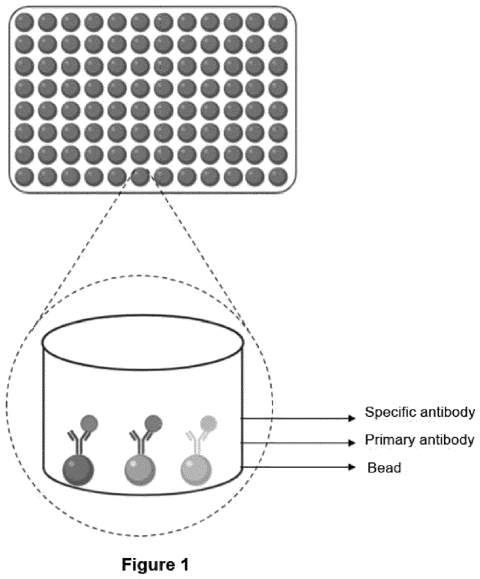

The panel of biomarkers is of special interest not only for the diagnosis, but also for the prognosis of the disease. The method of the invention is based on the use of said panel of biomarkers for the diagnosis and prognosis of AD and represents a substantial improvement with respect to the traditionally used methods (Figure 1).

Therefore, in a first aspect, the invention relates to an *in vitro* method for the diagnosis or prognosis of AD in a subject, the method comprises: a) measuring the expression levels of a panel of biomarkers in a biological sample from the subject; b) comparing the expression levels of each biomarker determined in a) with the respective reference levels; and c) performing a statistical analysis to score a final value X from 1 to 10, said value X indicating the subject's risk of developing the disease, wherein the panel of biomarkers comprises: (i) Beta-amyloid 1-40 (βA40); Beta-amyloid 1-42 (βA42); Cell surface glycoprotein MUC18 (MUC18); Apolipoprotein C2 (APOC2); Apolipoprotein A1 (APOA1); Albumin (ALBU); and retinol-binding protein 4 (RET4); and (ii) total Tau protein (tTau); phosphorylated Tau protein (pTau; Thr181); Apolipoprotein E (APOE); Clusterin (CLUS); SPARC (SPRC); Chitinase 3-like-1 (CH3L1/YKL40); and Malate dehydrogenase 1 (MDHC); and wherein: 1 ≤ X <5 indicates that there is no significant risk of developing AD; 5 ≤ X <7.5 indicates a slight risk of developing AD; and X ≥ 7.5 indicates a high risk of developing AD.

Alzheimer's disease is the most common cause of dementia, an acquired progressive cognitive deterioration that is sufficient to affect daily activities. Current estimations suggest that 44 million people now live with dementia worldwide, and this figure is expected to triple in 2050 as the population ages.

AD represent 50% to 75% of diagnosed dementias, being mainly a condition of the last stage of life. The main characteristics of the pathology are amyloid plaques and neurofibrillary tangles (NFTs). Furthermore, neuropil threads, associated dystrophic neurites, astrogliosis, and microglial activation are observed, and cerebral amyloid angiopathy often coexists.

The disease is diagnosed based on clinical evaluation, and particularly on clinical interview with the patient and an informant, and a specific cognitive and physical examination. Structural imaging using computed tomography or, ideally, magnetic resonance, is recommended for all patients investigated for cognitive deterioration in order to exclude structural anomalies and provide information of a positive diagnosis. PET imaging of amyloid is also now available for clinical diagnosis. However, there is still a need to identify reliable biomarkers for the effective diagnosis and prognosis of AD.

The use of biomarkers for the diagnosis of AD would have several advantages. An ideal marker would allow diagnosis in a very early stage of the disease, before degeneration related to the disease is observed in brain imaging and neuropathological tests. This, in turn, would allow starting treatment as soon as possible and monitoring the development of the disease, potentially allowing the efficacy of new therapies to be tested.

The term "diagnosis", as it is used herein, includes providing any information related to the existence or presence, nonexistence or absence, or probability of the disorder in a patient. It further includes providing information about the type or classification of the disorder or symptoms that are experienced or may be experienced in relation to the disorder. This can include, for example, the diagnosis of the severity of the disorder.

The term "prognosis", as it is used herein, refers to determining the probable outcome or course of a disease, particularly the possibilities of recovery or recurrence of said disease.

Methods for determining the expression levels of a gene are widely known in recent developments and any of said methods can be used in the context of the present invention. Determination of the expression levels of genes encoding protein biomarkers used in the method of the invention may comprise measuring the cDNA level, the mRNA level, and/or the level of the protein encoded by said gene. By way of non-limiting example, the mRNA levels of said gene can be quantified by means of using conventional methods, for example, methods comprising the amplification of mRNA and the quantification of the amplification product of said mRNA, such as electrophoresis and staining, or as an alternative, by means of Southern blot analysis and the use of suitable probes, Northern blot analysis and the use of probes specific for the mRNA of the gene of interest (gene encoding the protein of interest) or of the corresponding cDNA thereof, nuclease S1 mapping, hybridization, RT-Q-PCR, microarrays, and RNA sequencing, etc.

If the expression of the gene encoding the protein of interest is to be quantified by measuring protein levels, the biological sample isolated from the subject must be treated to extract the proteins. Methods for extracting or isolating proteins are known to those skilled in the art and are commercially available. The levels of the gene encoding the protein of interest can be quantified by means of any conventional method that allows detecting and quantifying said protein in a sample from a subject. By way of non-limiting example, the levels of said protein can be quantified, for example, by means of using antibodies capable of binding specifically to the protein of interest and subsequently quantifying the complexes formed.

In a preferred embodiment of the present invention, the expression levels of the gene encoding the protein of interest are quantified by measuring the protein levels.

The expression "reference level" refers to the expression levels of the gene encoding the protein biomarker of interest in a sample from a control subject, in other words, a healthy person who does not suffer from AD who is of the same age and sex as the subject.

The expression levels of the biomarkers of interest for a given subject and a control subject can be obtained at one or more time points to follow the progress of AD. The expression "time point", as it is used herein, corresponds to the moment in which the samples to be analyzed are obtained. The method of the invention can also be carried out at one or more time points.

In some embodiments, when the expression levels of the biomarkers of interest for a given subject and a control subject are obtained at more than one time point, said time points are selected from the group consisting of: zero, one, two, three, four, five, six, seven, eight, nine, ten, eleven, and twelve months, wherein the time point zero is understood to mean the first time the method of the invention is carried out. In said embodiments, the method of the invention can be carried out at the same time points. Alternatively, the method of the invention can be carried out at a later time point. In that case, samples can be obtained before that time and suitably stored.

The expression levels can be determined using any type of biological sample from the subject. As it is used herein, the expression "biological sample" refers to any material comprising a nucleic acid. Examples of biological samples include, but are not limited to, a biopsy sample, a tissue (for example, brain tissue), cell, or fluid (for example, serum, saliva, semen, sputum, CSF, tears, mucus, sweat, brain extracts, and the like), and blood (for example, PBMC (peripheral blood mononuclear cells) such as neutrophils, monocytes).

In some embodiments of the present invention, the biological sample is a fluid. In a preferred embodiment of the invention, the biological sample is CSF.

As it is used herein, the term "subject" refers to human and non-human animals, such as veterinary subjects. The expression "non-human animal" includes all vertebrates, for example, mammals and non-mammals. In some embodiments, the subject is a mammal. In a preferred embodiment, the subject is a human subject.

The panel of biomarkers of the method of the invention comprises: (i) βA40; βA42; MUC18; APOC2; APOA1; ALBU; RET4; and (ii) tTau; pTau (Thr181); APOE; CLUS; SPARC; MDHC; and CH3L1.

In some embodiments of the invention, the panel of biomarkers of the method of the invention consists of: (i) βA40; βA42; MUC18; APOC2; APOA1; ALBU; RET4, and (ii) tTau; pTau (Thr181); APOE; CLUS; SPARC; MDHC; and CH3L1.

The panel of biomarkers of the method of the invention may further comprise at least one biomarker selected from the group comprising: (iii) Neural pentraxin 2 (NPTX2); Nerve growth factor inducible (VGF); Proprotein convertase subtilisin/kexin type 1/proSAAS (PCSK1N) inhibitor; ceruloplasmin (CERU); and angiotensin (ANGT); and (iv) Serpin family F member 1/pigment epithelium-derived factor (PEDF); Spondin 1 (SPON1); Complement C3 (CO3); Alpha-2-macroglobulin (A2MG); Alpha-1-antitrypsin family A member 1/Serpin (A1AT); Osteopontin (OSTP); Fructose-biphosphate aldolase A (ALDOA); and SPARC-related modular calcium-binding protein 1 (SMOC1).

In some embodiments of the invention, the panel of biomarkers of the method of the invention comprises: (v) βA40; βA42; MUC18; APOC2; APOA1; ALBU; RET4; NPTX2; VGF; PCSK1N; CERU; and ANGT; and (vi) tTau; pTau (Thr181); APOE; CLUS; SPARC; CH3L1; MDHC; PEDF; SPON1; CO3; A2MG; A1AT; OSTP; ALDOA; and SMOC1.

In some embodiments of the invention, the panel of biomarkers of the method of the invention consists of: (v) βA40; βA42; MUC18; APOC2; APOA1; ALBU; RET4; NPTX2; VGF; PCSK1N; CERU; and ANGT; and (vi) tTau; pTau (Thr181); APOE; CLUS; SPARC; CH3L1; MDHC; PEDF; SPON1; CO3; A2MG; A1AT; OSTP; ALDOA; and SMOC1.

βA40 (UniProt P05067-PRO_0000000093, entry version 294) and βA42 (UniProt P05067-PRO_0000000092, entry version 294) are the main components of extracellular amyloid plaques which characterize AD. They are produced by the proteolysis of the transmembrane beta-amyloid precursor protein (APP) which works as a cell surface receptor and performs physiological functions on the surface of neurons which are important for neurite growth, neuronal adhesion, and axonogenesis. Beta-amyloid peptides bind to lipoproteins and apolipoproteins E and J in the CSF and to HDL particles in the plasma, inhibiting the metal-catalyzed oxidation of lipoproteins. βA42 can activate mononuclear phagocytes in the brain and cause inflammatory responses.

MUC18 (UniProt P43121, entry version 185) plays a role in cell adhesion and in the cohesion of the endothelial monolayer in the intercellular junctions of vascular tissue. The role of MUC18 as a possible biomarker for AD has still not been investigated in detail, and its possible relation to the disease has only been suggested recently (43).

APOC2 (UniProt P02655, entry version 218) is a key component of high-density lipoproteins (HDLs) and plays an important role in lipoprotein metabolism as lipoprotein lipase activator. Interestingly, changes in lipid metabolism can possibly be related to cognitive state in AD, so the use of APOC2 as a biomarker may be particularly relevant for determining the progression of the disease.

APOA1 (UniProt P02647, entry version 254) participates in the reverse transport of cholesterol from tissues to the liver for its excretion, promoting the exit of cholesterol from tissues and acting as a cofactor of lecithin cholesterol acyltransferase (LCAT). It has been identified as a strong and specific binding member of the full-length amyloid precursor protein APP (APPfl). Particularly, APP is the source of neurotoxic beta-amyloid peptide (betaA or βA) associated with Alzheimer's disease. ApoA-I binds to betaA and inhibits the formation of betaA beta sheets, and also attenuates betaA-induced cytotoxicity (44). In fact, high APOA1 levels may be related to lower risks of developing dementia (45).

ALBU (UniProt P02768, entry version 272) is the most abundant protein in human blood plasma. It transports βA, hormones, and fatty acids, among others, and helps to maintain osmotic pressure. The oxidation levels of the protein are higher in patients with AD, which suggests the possible role thereof as a biomarker (46).

RET4 (UniProt P02753, entry version 228) is a retinol-binding protein which mediates retinol transport in blood plasma. Therefore, it plays a key role in retinoic acid signaling, as well as in insulin metabolism, both of which have been demonstrated as being related to AD (47). Low expression levels have been observed in patients with AD (15).

NPTX2 (UniProt P47972, entry version 172) is a member of a 'long' neuronal pentraxin family. Its downregulation is closely related to cognitive deterioration and possibly associated with both, neuronal degradation and synaptic loss (48).

VGF (UniProt O15240, entry version 135) is a secreted neuronal and endocrine protein whose expression is induced by BDNF. The protein is processed into several bioactive peptides which participate in memory formation and neuroprotection. Biomarker studies have identified reduced levels of VGF-derived peptides in the cerebrospinal fluid of patients with AD (49).

PCSK1N (UniProt Q9UHG2, entry version 142) belongs to the granin protein family and is expressed almost exclusively in neurons and endocrine cells. Reduced levels of PCSK1N in the CSF of patients with AD can be related to a greater brain retention of this protein in plaques and other aggregates (50).

CERU (UniProt P00450, entry version 225) is the main copper-transporting protein in plasma and also acts by limiting free iron concentrations, thereby playing an important antioxidant role in serum. Copper is an essential cofactor for many enzymes, including cytochromes, but it is toxic in free form. Most serum copper is transported bound to ceruloplasmin. Interestingly, it has been described that a decrease in active CERU in the CSF of patients with AD, associated with an increase in the reserve of copper not sequestrated by this protein, may play a role in the neurodegenerative process (51).

ANGT (UniProt P01019, entry version 236) is an essential component of the renin-angiotensin system (RAS) and a potent regulator of blood pressure, and homeostasis of body fluid and electrolyte. The relationship between the development of cardiovascular risk factors and the pathogenesis of AD has been established previously, which suggests the role of ANGT in age-associated cognitive deterioration (52).

CLUS or APOJ (UniProt P10909, entry version 232) is a multifunctional glycoprotein that has been implicated in several physiological and pathological states, including Alzheimer's disease (AD), among others. It is involved in pathways that are common to several diseases such as cell death and survival, oxidative stress, and proteotoxic stress. The relationship of clusterin with beta-amyloid peptide (βA) has been of great interest in the AD field, including the apparent role of clusterin in altering βA aggregation and/or elimination. In addition, clusterin has more recently been identified as a mediator of βA toxicity, as demonstrated by the neuroprotective effect of gene inactivation and reduced CLU expression in neurons generated from rodent and human iPSCs. CLU is also the third most important genetic risk factor for late onset AD and several variants in CLU have been identified (53).

SPARC (UniProt P09486, entry version 218) participates in tissue development and repair by regulating cell adhesion, proliferation, metastasis, and growth factor signaling which affects the extracellular matrix (ECM) and cell-cell interactions. In the brain, the expression of SPARC is restricted to microglia and subcortical astrocytes, and a role for SPARC in neuroinflammation has been suggested. Specifically, high levels of SPARC have been demonstrated in AD brain, where it colocalizes with βA protein deposits. It has been proposed that SPARC contributes to brain inflammation and subsequent tissue repair (54).

CH3L1 (UniProt P36222, entry version 191) is expressed in the CNS by microglia and astrocytes and constitutes one of the most interesting potential biomarkers in AD. Increased expression of CH3L1 is found in human brains of individuals with pathologically confirmed AD, implicating CH3L1 in the neuroinflammatory response to βA deposition (55).

MDHC (UniProt P40925, entry version 207) is an enzyme that reversibly catalyzes the oxidation of malate to oxaloacetate by means of reducing NAD+ to NADH. Increased activity of this protein has been demonstrated in the brain of patients with AD (56).

PEDF (UniProt P36955, entry version 207) is a unique neurotrophic protein, and its expression decreases with aging. Elevated βA42 levels have been observed in mice in which the PEDF gene is inactivated (KO); in addition, PEDF notably reduced cognitive deterioration in the Morris water maze (MWM) and significantly reduced βA42 in SAMP8 mice. Therefore, it has been suggested that PEDF downregulates βA42 and that aging-linked PEDF deficiency may play a crucial role in the development of AD (57).

SPON1 (UniProt Q9HCB6, entry version 157) is a cell adhesion protein that promotes spinal cord and sensory neuronal cell binding and neurite growth *in vitro.* It binds to BACE at the amyloid precursor protein (APP) binding site and blocks the start of amyloidogenesis (58).

COS (UniProt P01024, entry version 248) plays a main role in the activation of the complement system. Its processing by C3 convertase is the main reaction in both classical and alternative complement pathways. After activation, C3b can covalently bind, through its reactive thioester, to cell surface carbohydrates or immune aggregates. It has been proposed that it may be involved in the recruitment and activation of microglia in βA fibrillar deposit regions (59).

A2MG (UniProt P01023, entry version 227) is a major component of the innate immune system; it works as a pan-protease inhibitor and a chaperone protein. Previous evidence indicates that A2MG is capable of binding to aggregation-prone and poorly folded proteins of the patient and may play an important role in the pathogenesis of AD. In fact, A2MG is associated with preclinical AD, reflecting neuronal injury early in the course of the disease, and may respond to tau phosphorylation in the brain through the RCAN1-calcineurin pathway (60).

A1AT (UniProt P01009, entry version 267) is a serine protease inhibitor involved in neurodegeneration and neuroinflammation. Elevated levels of this protein have been observed in CSF in AD, which suggests that its overexpression may damage neural functioning (61).

OSTP (UniProt P10451, entry version 220) is a matricellular immunomodulatory cytokine highly expressed by myelomonocytic cells, and is known to regulate the immune cell migration, communication, and response to brain injury, being involved in inflammatory and degenerative processes of the nervous system. In common neurodegenerative diseases, such as Parkinson's and Alzheimer's disease, OPN appears to have a dual effect, triggering neuronal toxicity and death in some contexts and working as a neuroprotectant in others (62). The protein also plays an essential role in modulating both the immune profile of macrophages and their capacity to resist pathogenic forms of βA (63).

ALDOA (UniProt P04075, entry version 241) is a glycolytic enzyme that has been characterized as a common autoantigen in AD, which suggests its role as a novel target for possible immune modulation (64).

SMOC1 (UniProt Q9H4F8, entry version 173) is found in basement membranes and is thought to regulate growth factors that stimulate tissue growth and development throughout the body. SMOC1 levels in postmortem brain are highly correlated with the pathology of AD even in the preclinical stage of the disease, which indicates that SMOC1 levels in CSF reflect underlying AD-specific brain pathology (65).

In the method of the invention, the expression levels of the biomarkers of (i), (iii), and (v) decrease with the progression of AD. Therefore, lower expression levels of said biomarkers compared to a reference level can indicate a risk of the subject to develop AD and/or a worse prognosis of the disease.

In the method of the invention, the expression levels of the biomarkers of (ii), (iv), and (vi) increase with the progression of AD. Therefore, higher expression levels of said biomarkers compared to a reference level can indicate a risk of the subject to develop AD and/or a worse prognosis of the disease.

The method of the invention comprises performing a statistical analysis to score a final value X from 1 to 10, which will indicate the subject's risk of developing the disease in the following manner:
1 ≤ X <5: no significant risk of developing AD;
5 ≤ X <7.5: slight risk of developing AD; and
X ≥ 7.5: high risk of developing AD.

In some embodiments of the invention, one skilled in the art can conclude that the subject is at risk of developing AD when the expression levels of seven or more biomarkers show a change of at least 2.5 folds compared to the reference levels. In these particular embodiments, a value of 0.8 is added to the score X calculated.

In some embodiments of the invention, one skilled in the art can conclude that the subject is at risk of developing AD when the expression levels of thirteen or more biomarkers show a change of at least 1.5 folds compared to the reference levels. In these particular embodiments, a value of 0.4 is added to the score X calculated.

It is understood that changes of at least 1.5 and 2.5 folds refer to (a) a decrease in the expression levels for those biomarkers selected from the group consisting of βA40, βA42, MUC18, APOC2, APOA1, ALBU, RET4, NPTX2, VGF, PCSK1N, CERU, and ANGT; or (b) an increase in the expression levels for those biomarkers selected from the group consisting of tTau, pTau, APOE, CLUS, SPRC, CH3L1, MDHC, PEDF, SPON1, CO3, A2MG, A1AT, OSTP, ALDOA, and SMOC1.

In some embodiments of the invention, the statistical analysis and diagnosis are performed by means of a predictive algorithm. Blocking factors relating to patients' characteristics can be included in the analysis to correct false positives or negatives. In addition, a factor analysis can be performed to reduce the dimension of the number of variables according to sample size. Therefore, univariate and multivariate logistic regression models can be calculated.

The expression "biomarker of interest", as it is used herein, refers to any of the biomarkers selected from the group consisting of: βA40, βA42, MUC18, APOC2, APOA1, ALBU, RET4, NPTX2, VGF, PCSK1N, CERU, ANGT, tTau, pTau, APOE, CLUS, SPRC, CH3L1, MDHC, PEDF, SPON1, CO3, A2MG, A1AT, OSTP, ALDOA, and SMOC1.

In some embodiments of the invention, the biomarkers are detected by means of using one or more binding agents including, among others, antibodies specific for each biomarker. In other embodiments, the biomarkers are detected using at least one autoantibody specific for each biomarker.

The term "antibody" is understood to mean a polypeptide including at least one immunoglobulin light chain or heavy chain variable region which specifically recognizes and binds to an epitope of an antigen, for example, the biomarkers for use thereof in the method of the invention. Antibodies are composed of a heavy chain and a light chain, each of which has a variable region, referred to as heavy chain variable region (VH) and light chain variable region (VL). Overall, the VH region and the VL region are responsible for binding to the antigen recognized by the antibody. The antibodies of the present disclosure include those specific for the biomarkers of the panel for use thereof in the invention.

The term "antibody" includes intact immunoglobulins, as well as variants and parts thereof, such as Fab' fragments, F(ab)'2 fragments, single-chain Fv proteins ("scFv"), and disulfide-stabilized Fv proteins ("dsFv"). An scFv protein is a fusion protein in which a light chain variable region of an immunoglobulin and a heavy chain variable region of an immunoglobulin are attached by a linker, whereas in dsFv, the chains have been mutated to introduce a disulfide bond to stabilize the chain association. The term also includes genetically modified forms such as chimeric antibodies (for example, humanized murine antibodies), heteroconjugated antibodies (such as diabodies or bispecific antibodies).

Antibodies can be prepared using any of the methods known to one skilled in the art. For example, they can be prepared by means of immunizing an animal with the protein to be inhibited.

The term "autoantibody", as it is used herein, refers to antibodies which react with own molecules found in healthy individuals. Natural autoantibodies show moderate affinity for autoantigens and provide a first line of defense against infections. High-affinity somatically mutated IgG autoantibodies reflect a pathological process through which hemostatic pathways related to cellular excretion, antigen receptor signaling, or cell effector functions are altered. In some autoimmune disorders, autoantibodies can be present before the onset of the disease, show significant specificity, and serve as biomarkers that provide opportunity for diagnosis and therapeutic intervention.

In some embodiments, the binding agents or autoantibodies can be fixed on a solid support including, but is not limited to, a membrane, a magnetic bead, a microplate, or an array.

In some embodiments of the invention, the binding agents or autoantibodies are fixed on a microplate.

In a preferred embodiment of the invention, the binding agents are antibodies specific for the biomarkers of the panel and the solid support is a microplate.

In some embodiments, the method comprises simultaneously determining the expression levels of each of the biomarkers in a microplate using a panel of antibodies, each of which is specific for one of the biomarkers. Individual determination of the expression levels of each of the biomarkers is achieved by placing each antibody in a unique targetable location, thereby allowing a separate assay reading for each individual biomarker in the samples, as well as readings for any selected combination of biomarkers.

By way of non-limiting example, simultaneous determination of the biomarkers can be achieved using the Luminex technology. This technology is based on the use of a mixtures of color-labeled beads previously coated with analyte-specific capture antibodies. Once the antibodies bind to the analytes of interest, biotinylated detection antibodies specific for the analytes of interest and phycoerythrin (PE)-conjugated streptavidin are added to form an antibody-antigen sandwich, that binds to biotinylated detection antibodies. The beads are read on a dual laser flow detection instrument, such as the Luminex 200^{™} or FlexMap^{®} analyzer, or the Luminex MAGPIX^{®} analyzer.

A second aspect of the invention relates to the *in vitro* use of a kit comprising reagents for measuring the expression levels of a panel of biomarkers in a biological sample from a subject according to the method of the invention.

The kit can include, but are not limited to, the biomarkers of interest and/or reagents that bind specifically to the individual markers, such as the binding agents, for example, antibodies, that can suitably be used for the diagnosis of patients having AD symptoms. The kit can further include a solid support for the binding agents. In addition, the kit also can include instructions for use.

Unless defined otherwise, all technical and scientific terms used herein have the same meaning as that commonly understood by one skilled in the art to which the present invention belongs. Methods and materials similar or equivalent to those described herein can be used to put the present invention into practice. Throughout the description and claims, the word "comprise" and its variations are not intended to exclude other technical features, additives, components or steps. Additional objects, advantages, and features of the invention will be apparent to those skilled in the art upon examining the description or may be learned by means of putting the invention into practice. The following examples are provided by way of illustration and are not intended to be limiting of the present invention.

### BRIEF DESCRIPTION OF THE DRAWINGS

Figure 1. Illustrative diagram of the method of the invention.

### EXAMPLES

### Materials

CSF samples. CSF samples are provided by BT-CIEN biobank (Madrid) and comprise at least samples from (i) ten individuals diagnosed with AD and (ii) six healthy individuals, in other words, not diagnosed with AD. The samples are diluted in the assay buffer of choice (kits 1 to 4 as described below, according to manufacturer's instructions).

Protein detection and quantification kits. The following kits are used:
1. HNABTMAG-68K-04 (Merck): for the detection and quantification of Aβ40, Aβ42, pTau (Thr181), and Tau (total).
2. APOMAG-62K-03 (Merck): for the detection and quantification of APOA1, APOC2, and APO E.
3. SPR1937 (Merck): for the detection and quantification of CLUS, MUC18, ALBU, and MDHC.
4. SPR1938 (Merck): for the detection and quantification of SPRC, CH3L1, and RET4.

### Methods

The kits are used according to manufacturer's instructions.

Biomarkers are detected and quantified using the multi-analyte profiling (xMAP) technology and Luminex equipment according to manufacturer's instructions. Luminex's xMAP technology uses fluorescence-labeled magnetic beads conjugated with specific antibodies which recognize a protein of interest. The information collection system is based on a reader (MAGPIX) that detects both the labeled bead and the protein, thereby allowing the analysis of several proteins in the same sample, which ultimately generates the specific protein profile.

The assays are performed in 96-well plates, 16 of which are used for standards. Before the analyses, several optimization tests are performed using different antibody and sample dilutions.

All the samples are analyzed in duplicate and each of the plates includes a positive interplate control. Median fluorescent intensity (MFI) values are exported and the characteristics of individual samples are confirmed by calculating the mean, the standard deviation (SD), and the coefficient of variation (% of CV) of the MFI readings.

### Data analysis

The data obtained are statistically analyzed to determine the relationship between the concentrations of the panel of biomarkers taken into consideration herein and the dependent variable, that is, the development of Alzheimer's disease.

The concentration of each biomarker is determined by the fluorescence intensity thereof in the sample. Furthermore, blocking factors relating to patients' characteristics are included in the analysis to correct false results. If necessary, a factor analysis is performed to reduce the dimension of the number of variables according to sample size. Univariate and multivariate logistic regression models are thus applied, the adjusted odd ratios obtained from these models allow quantifying the effect of each biomarker and the direction of said effect. The diagnostic capacity of these models is verified by means of ROC curves, showing the sensitivity and specificity values for the cut-off points, providing the probability of development of the disease for each patient.

### Results

The CSF samples obtained from subjects with AD shows increased levels of pTau, tTau, APOE, CLUS, MDHC, SPRC, and CH3L1 and decreased levels of βA40, βA42, APOA1, APOC2, MUC18, ALBU, and RET4. The expression levels of all these proteins in said samples show a change of at least 1.5 when compared with the corresponding expression levels in control subjects, and the average value of score X in the samples is at least 5.

### REFERENCES

1. Blennow K, Zetterberg H. Fluid biomarker-based molecular phenotyping of Alzheimer's disease patients in research and clinical settings. Prog Mol Biol Transl Sci. 2019; 168:3-23.
2. Khoonsari PE, Shevchenko G, Herman S, Remnestal J, Giedraitis V, Brundin R, et al. Improved Differential Diagnosis of Alzheimer's Disease by Integrating ELISA and Mass Spectrometry-Based Cerebrospinal Fluid Biomarkers. Journal of Alzheimer's disease: JAD. 2019;67(2):639-51.
3. Khoonsari PE, Haggmark A, Lonnberg M, Mikus M, Kilander L, Lannfelt L, et al. Analysis of the Cerebrospinal Fluid Proteome in Alzheimer's Disease. PloS one. 2016;11(3):e0150672.
4. Alzate O, Osorio C, DeKroon RM, Corcimaru A, Gunawardena HP. Differentially charged isoforms of apolipoprotein E from human blood are potential biomarkers of Alzheimer's disease. Alzheimers Res Ther. 2014;6(4):43.
5. Chakrabarti A, Chatterjee A, Sengupta MB, Chattopadhyay P, Mukhopadhyay D. Altered levels of amyloid precursor protein intracellular domain-interacting proteins in Alzheimer disease. Alzheimer Dis Assoc Disord. 2014;28(3):283-90.
6. Castano EM, Roher AE, Esh CL, Kokjohn TA, Beach T. Comparative proteomics of cerebrospinal fluid in neuropathologically-confirmed Alzheimer's disease and non-demented elderly subjects. Neurol Res. 2006;28(2):155-63.
7. Puchades M, Hansson SF, Nilsson CL, Andreasen N, Blennow K, Davidsson P. Proteomic studies of potential cerebrospinal fluid protein markers for Alzheimer's disease. Brain Res Mol Brain Res. 2003;118(1-2):140-6.
8. Davidsson P, Westman-Brinkmalm A, Nilsson CL, Lindbjer M, Paulson L, Andreasen N, et al. Proteome analysis of cerebrospinal fluid proteins in Alzheimer patients. Neuroreport. 2002;13(5):611-5.
9. Sathe G, Na CH, Renuse S, Madugundu AK, Albert M, Moghekar A, et al. Quantitative Proteomic Profiling of Cerebrospinal Fluid to Identify Candidate Biomarkers for Alzheimer's Disease. Proteomics Clin Appl. 2019;13(4):e1800105.
10. Perrin RJ, Craig-Schapiro R, Malone JP, Shah AR, Gilmore P, Davis AE, et al. Identification and validation of novel cerebrospinal fluid biomarkers for staging early Alzheimer's disease. PloS one. 2011;6(1):e16032.
11. Wang HF, Tan L, Cao L, Zhu XC, Jiang T, Tan MS, etal. Application of the IWG-2 Diagnostic Criteria for Alzheimer's Disease to the ADNI. Journal of Alzheimer's disease: JAD. 2016;51(1):227-36.
12. Spellman DS, Wildsmith KR, Honigberg LA, Tuefferd M, Baker D, Raghavan N, et al. Development and evaluation of a multiplexed mass spectrometry based assay for measuring candidate peptide biomarkers in Alzheimer's Disease Neuroimaging Initiative (ADNI) CSF. Proteomics Clin Appl. 2015;9(7-8):715-31.
13. Ringman JM, Schulman H, Becker C, Jones T, Bai Y, Immermann F, et al. Proteomic changes in cerebrospinal fluid of presymptomatic and affected persons carrying familial Alzheimer disease mutations. Arch Neurol. 2012;69(1):96-104.
14. Holtta M, Minthon L, Hansson O, Holmen-Larsson J, Pike I, Ward M, et al. An integrated workflow for multiplex CSF proteomics and peptidomics-identification of candidate cerebrospinal fluid biomarkers of Alzheimer's disease. Journal of proteome research. 2015;14(2):654-63.
15. Jung SM, Lee K, Lee JW, Namkoong H, Kim HK, Kim S, etal. Both plasma retinol-binding protein and haptoglobin precursor allele 1 in CSF: candidate biomarkers for the progression of normal to mild cognitive impairment to Alzheimer's disease. Neurosci Lett. 2008;436(2):153-7.
16. Finehout EJ, Franck Z, Choe LH, Relkin N, Lee KH. Cerebrospinal fluid proteomic biomarkers for Alzheimer's disease. Ann Neurol. 2007;61(2): 120-9.
17. Duits FH, Brinkmalm G, Teunissen CE, Brinkmalm A, Scheltens P, Van der Flier WM, et al. Synaptic proteins in CSF as potential novel biomarkers for prognosis in prodromal Alzheimer's disease. Alzheimers Res Ther. 2018;10(1):5.
18. Brinkmalm G, Sjodin S, Simonsen AH, Hasselbalch SG, Zetterberg H, Brinkmalm A, et al. A Parallel Reaction Monitoring Mass Spectrometric Method for Analysis of Potential CSF Biomarkers for Alzheimer's Disease. Proteomics Clin Appl. 2018;12(1).
19. Skillback T, Mattsson N, Hansson K, Mirgorodskaya E, Dahlen R, van der Flier W, et al. A novel quantification-driven proteomic strategy identifies an endogenous peptide of pleiotrophin as a new biomarker of Alzheimer's disease. Scientific reports. 2017;7(1):13333.
20. Hendrickson RC, Lee AY, Song Q, Liaw A, Wiener M, Paweletz CP, et al. High Resolution Discovery Proteomics Reveals Candidate Disease Progression Markers of Alzheimer's Disease in Human Cerebrospinal Fluid. PloS one. 2015;10(8):e0135365.
21. Wijte D, McDonnell LA, Balog CI, Bossers K, Deelder AM, Swaab DF, et al. A novel peptidomics approach to detect markers of Alzheimer's disease in cerebrospinal fluid. Methods. 2012;56(4):500-7.
22. Jahn H, Wittke S, Zurbig P, Raedler TJ, Arlt S, Kellmann M, et al. Peptide fingerprinting of Alzheimer's disease in cerebrospinal fluid: identification and prospective evaluation of new synaptic biomarkers. PloS one. 2011;6(10):e26540.
23. Simonsen AH, McGuire J, Hansson O, Zetterberg H, Podust VN, Davies HA, et al. Novel panel of cerebrospinal fluid biomarkers for the prediction of progression to Alzheimer dementia in patients with mild cognitive impairment. Arch Neurol. 2007;64(3):366-70.
24. Simonsen AH, McGuire J, Podust VN, Davies H, Minthon L, Skoog I, et al. Identification of a novel panel of cerebrospinal fluid biomarkers for Alzheimer's disease. Neurobiol Aging. 2008;29(7):961-8.
25. Selle H, Lamerz J, Buerger K, Dessauer A, Hager K, Hampel H, et al. Identification of novel biomarker candidates by differential peptidomics analysis of cerebrospinal fluid in Alzheimer's disease. Comb Chem High Throughput Screen. 2005;8(8):801-6.
26. Carrette O, Demalte I, Scherl A, Yalkinoglu O, Corthals G, Burkhard P, et al. A panel of cerebrospinal fluid potential biomarkers for the diagnosis of Alzheimer's disease. Proteomics. 2003;3(8):1486-94.
27. Wang J, Cunningham R, Zetterberg H, Asthana S, Carlsson C, Okonkwo O, et al. Label-free quantitative comparison of cerebrospinal fluid glycoproteins and endogenous peptides in subjects with Alzheimer's disease, mild cognitive impairment, and healthy individuals. Proteomics Clin Appl. 2016;10(12):1225-41.
28. Abdi F, Quinn JF, Jankovic J, Mclntosh M, Leverenz JB, Peskind E, et al. Detection of biomarkers with a multiplex quantitative proteomic platform in cerebrospinal fluid of patients with neurodegenerative disorders. Journal of Alzheimer's disease: JAD. 2006;9(3):293-348.
29. Higginbotham L, Ping L, Dammer EB, Duong DM, Zhou M, Gearing M, et al. Integrated proteomics reveals brain-based cerebrospinal fluid biomarkers in asymptomatic and symptomatic Alzheimer's disease. Sci Adv. 2020;6(43).
30. Bader JM, Geyer PE, Muller JB, Strauss MT, Koch M, Leypoldt F, etal. Proteome profiling in cerebrospinal fluid reveals novel biomarkers of Alzheimer's disease. Mol Syst Biol. 2020;16(6):e9356.
31. Maarouf CL, Andacht TM, Kokjohn TA, Castano EM, Sue LI, Beach TG, et al. Proteomic analysis of Alzheimer's disease cerebrospinal fluid from neuropathologically diagnosed subjects. Curr Alzheimer Res. 2009;6(4):399-406.
32. Heywood WE, Galimberti D, Bliss E, Sirka E, Paterson RW, Magdalinou NK, et al. Identification of novel CSF biomarkers for neurodegeneration and their validation by a high-throughput multiplexed targeted proteomic assay. Mol Neurodegener. 2015; 10:64.
33. Manral P, Sharma P, Hariprasad G, Chandralekha, Tripathi M, Srinivasan A. Can apolipoproteins and complement factors be biomarkers of Alzheimer's disease? Curr Alzheimer Res. 2012;9(8):935-43.
34. Skillbäck T, Mattsson N, Hansson K, Mirgorodskaya E, Dahlén R, van der Flier W, et al. A novel quantification-driven proteomic strategy identifies an endogenous peptide of pleiotrophin as a new biomarker of Alzheimer's disease. Scientific reports. 2017;7(1):13333-.
35. Whelan CD, Mattsson N, Nagle MW, Vijayaraghavan S, Hyde C, Janelidze S, et al. Multiplex proteomics identifies novel CSF and plasma biomarkers of early Alzheimer's disease. Acta Neuropathol Commun. 2019;7(1):169.
36. Paterson RW, Heywood WE, Heslegrave AJ, Magdalinou NK, Andreasson U, Sirka E, et al. A targeted proteomic multiplex CSF assay identifies increased malate dehydrogenase and other neurodegenerative biomarkers in individuals with Alzheimer's disease pathology. Transl Psychiatry. 2016;6(11):e952.
37. Wildsmith KR, Schauer SP, Smith AM, Arnott D, Zhu Y, Haznedar J, et al. Identification of longitudinally dynamic biomarkers in Alzheimer's disease cerebrospinal fluid by targeted proteomics. Mol Neurodegener. 2014;9:22.
38. Dayon L, Nunez Galindo A, Wojcik J, Cominetti O, Corthesy J, Oikonomidi A, et al. Alzheimer disease pathology and the cerebrospinal fluid proteome. Alzheimers Res Ther. 2018;10(1):66.
39. Hu WT, Chen-Plotkin A, Arnold SE, Grossman M, Clark CM, Shaw LM, et al. Novel CSF biomarkers for Alzheimer's disease and mild cognitive impairment. Acta neuropathologica. 2010;119(6):669-78.
40. Zhou M, Haque RU, Dammer EB, Duong DM, Ping L, Johnson ECB, et al. Targeted mass spectrometry to quantify brain-derived cerebrospinal fluid biomarkers in Alzheimer's disease. Clin Proteomics. 2020;17:19.
41. Wang H, Dey KK, Chen PC, Li Y, Niu M, Cho JH, et al. Integrated analysis of ultra-deep proteomes in cortex, cerebrospinal fluid and serum reveals a mitochondrial signature in Alzheimer's disease. Mol Neurodegener. 2020;15(1):43.
42. Maarouf CL, Kokjohn TA, Whiteside CM, Macias MP, Kalback WM, Sabbagh MN, et al. Molecular Differences and Similarities Between Alzheimer's Disease and the 5XFAD Transgenic Mouse Model of Amyloidosis. Biochem Insights. 2013;6:1-10.
43. Pedrero-Prieto CM, Garcia-Carpintero S, Frontinan-Rubio J, Llanos-Gonzalez E, Aguilera Garcia C, Alcain FJ, et al. A comprehensive systematic review of CSF proteins and peptides that define Alzheimer's disease. Clin Proteomics. 2020;17:21.
44. Koldamova RP, Lefterov IM, Lefterova MI, Lazo JS. Apolipoprotein A-I directly interacts with amyloid precursor protein and inhibits A beta aggregation and toxicity. Biochemistry. 2001;40(12):3553-60.
45. Paula-Lima AC, Tricerri MA, Brito-Moreira J, Bomfim TR, Oliveira FF, Magdesian MH, et al. Human apolipoprotein A-I binds amyloid-beta and prevents betaA-induced neurotoxicity. Int J Biochem Cell Biol. 2009;41(6):1361-70.
46. Costa M, Horrillo R, Ortiz AM, Perez A, Mestre A, Ruiz A, etal. Increased Albumin Oxidation in Cerebrospinal Fluid and Plasma from Alzheimer's Disease Patients. Journal of Alzheimer's disease: JAD. 2018;63(4):1395-404.
47. Ishii M, ladecola C. Metabolic and Non-Cognitive Manifestations of Alzheimer's Disease: The Hypothalamus as Both Culprit and Target of Pathology. Cell metabolism. 2015;22(5):761-76.
48. Swanson A, Wolf T, Sitzmann A, Willette AA. Neuroinflammation in Alzheimer's disease: Pleiotropic roles for cytokines and neuronal pentraxins. Behav Brain Res. 2018;347:49-56.
49. Wesenhagen KEJ, Teunissen CE, Visser PJ, Tijms BM. Cerebrospinal fluid proteomics and biological heterogeneity in Alzheimer's disease: A literature review. Critical reviews in clinical laboratory sciences. 2019:1-13.
50. Hoshino A, Helwig M, Rezaei S, Berridge C, Eriksen JL, Lindberg I. A novel function for proSAAS as an amyloid anti-aggregant in Alzheimer's disease. J Neurochem. 2014;128(3):419-30.
51. Capo CR, Arciello M, Squitti R, Cassetta E, Rossini PM, Calabrese L, et al. Features of ceruloplasmin in the cerebrospinal fluid of Alzheimer's disease patients. Biometals. 2008;21(3):367-72.
52. Kehoe PG. The Coming of Age of the Angiotensin Hypothesis in Alzheimer's Disease: Progress Toward Disease Prevention and Treatment? Journal of Alzheimer's disease: JAD. 2018;62(3):1443-66.
53. Foster EM, Dangla-Valls A, Lovestone S, Ribe EM, Buckley NJ. Clusterin in Alzheimer's Disease: Mechanisms, Genetics, and Lessons From Other Pathologies. Front Neurosci. 2019;13:164.
54. Strunz M, Jarrell JT, Cohen DS, Rosin ER, Vanderburg CR, Huang X. Modulation of SPARC/Hevin Proteins in Alzheimer's Disease Brain Injury. Journal of Alzheimer's disease: JAD. 2019;68(2):695-710.
55. Dhiman K, Blennow K, Zetterberg H, Martins RN, Gupta VB. Cerebrospinal fluid biomarkers for understanding multiple aspects of Alzheimer's disease pathogenesis. Cellular and molecular life sciences: CMLS. 2019;76(10):1833-63.
56. Bubber P, Haroutunian V, Fisch G, Blass JP, Gibson GE. Mitochondrial abnormalities in Alzheimer brain: mechanistic implications. Ann Neurol. 2005;57(5):695-703.
57. Huang M, Qi W, Fang S, Jiang P, Yang C, Mo Y, et al. Pigment Epithelium-Derived Factor Plays a Role in Alzheimer's Disease by Negatively Regulating Abeta42. Neurotherapeutics. 2018;15(3):728-41.
58. Hoe HS, Wessner D, Beffert U, Becker AG, Matsuoka Y, Rebeck GW. F-spondin interaction with the apolipoprotein E receptor ApoEr2 affects processing of amyloid precursor protein. Mol Cell Biol. 2005;25(21):9259-68.
59. Eikelenboom P, Veerhuis R. The role of complement and activated microglia in the pathogenesis of Alzheimer's disease. Neurobiol Aging. 1996;17(5):673-80.
60. Varma VR, Varma S, An Y, Hohman TJ, Seddighi S, Casanova R, et al. Alpha-2 macroglobulin in Alzheimer's disease: a marker of neuronal injury through the RCAN1 pathway. Molecular psychiatry. 2017;22(1):13-23.
61. Abu-Rumeileh S, Steinacker P, Polischi B, Mammana A, Bartoletti-Stella A, Oeckl P, et al. CSF biomarkers of neuroinflammation in distinct forms and subtypes of neurodegenerative dementia. Alzheimers Res Ther. 2019;12(1):2.
62. Carecchio M, Comi C. The role of osteopontin in neurodegenerative diseases. Journal of Alzheimer's disease: JAD. 2011;25(2):179-85.
63. Rentsendorj A, Sheyn J, Fuchs DT, Daley D, Salumbides BC, Schubloom HE, et al. A novel role for osteopontin in macrophage-mediated amyloid-beta clearance in Alzheimer's models. Brain, behavior, and immunity. 2018;67:163-80.
64. Mor F, Izak M, Cohen IR. Identification of aldolase as a target antigen in Alzheimer's disease. J Immunol. 2005;175(5):3439-45.
65. Zu F, Liu P, Wang H, Zhu T, Sun J, Sheng W, et al. Integrated analysis identifies a pathway-related competing endogenous RNA network in the progression of pancreatic cancer. BMC Cancer. 2020;20(1):958.

## Claims

1. An *in vitro* method for the diagnosis or prognosis of Alzheimer's disease (AD) in a subject, the method comprising:
a) measuring the expression levels of a panel of biomarkers in a biological sample from the subject;
b) comparing the expression levels of each biomarker determined in a) with the respective reference levels; and
c) performing a statistical analysis to score a final value X from 1 to 10, said value X indicating the subject's risk of developing the disease,
wherein the panel of biomarkers comprises:
(i) Beta-amyloid 1-40 (βA40); Beta-amyloid 1-42 (βA42); Cell surface glycoprotein MUC18 (MUC18); Apolipoprotein C2 (APOC2); Apolipoprotein A1 (APOA1); Albumin (ALBU); and retinol-binding protein 4 (RET4); and
(ii) total Tau protein (tTau); phosphorylated Tau protein (pTau; Thr181); Apolipoprotein E (APOE); Clusterin (CLUS); SPARC (SPRC); Chitinase 3-like-1 (CH3L1/YKL40); and malate dehydrogenase 1 (MDHC);
and wherein:
1 ≤ X <5 indicates that there is no significant risk of developing AD;
5 ≤ X <7.5 indicates a slight risk of developing AD; and
X ≥ 7.5 indicates a high risk of developing AD.

2. The method according to claim 1, wherein the panel of biomarkers further comprises at least one biomarker selected from the group comprising:
(iii) Neural pentraxin 2 (NPTX2); Nerve growth factor inducible (VGF); Proprotein convertase subtilisin/kexin type 1/proSAAS (PCSK1N) inhibitor; Ceruloplasmin (CERU); and Angiotensin (ANGT); and
(iv) Serpin family F member 1/pigment epithelium-derived factor (PEDF); Spondin 1 (SPON1); Complement C3 (CO3); Alpha-2-macroglobulin (A2MG); Alpha-1-antitrypsin family A member 1/Serpin; Osteopontin (OSTP); Fructose-biphosphate aldolase A (ALDOA); and SPARC-related modular calcium-binding protein 1 (SMOC1).

3. The method according to claim 1 or 2, wherein the panel of biomarkers comprises:
(v) βA40; βA42; MUC18; APOC2; APOA1; ALBU; RET4; NPTX2; VGF; CERU; PCSK1N; and ANGT; and
(vi) tTau; pTau (Thr181); APOE; CLUS; SPARC; CH3L1; MDHC; PEDF; SPON1; CO3; A2MG; A1AT; OSTP; ALDOA; and SMOC1.

4. The method according to any one of the preceding claims, wherein the panel of biomarkers consists of:
(v) βA40; βA42; MUC18; APOC2; APOA1; ALBU; RET4; NPTX2; VGF; CERU; PCSK1N; and ANGT; and
(vi) tTau; pTau (Thr181); APOE; CLUS; SPARC; CH3L1; MDHC; PEDF; SPON1; CO3; A2MG; A1AT; OSTP; ALDOA; and SMOC1.

5. The method according to any one of the preceding claims, wherein a multiple of variation equal to or greater than 2.5 in the expression levels of at least seven biomarkers indicates a risk of the subject developing AD.

6. The method according to any one of the preceding claims, wherein a multiple of variation equal to or greater than 1.5 in the expression levels of at least thirteen biomarkers indicates a risk of the subject developing AD.

7. The method according to any one of the preceding claims, wherein the biological sample is selected from the group consisting of: urine, blood, plasma, serum, saliva, and cerebrospinal fluid.

8. The method according to any one of the preceding claims, wherein the biomarkers are detected by means of a) using one or more binding agents or b) detecting at least one autoantibody specific for each biomarker.

9. The method according to claim 8, wherein the binding agents are fixed on a solid support including, but is not limited to, a membrane, a magnetic bead, a microplate, or an array.

10. The method according to any one of the preceding claims, wherein the method comprises simultaneously determining the expression levels of each of the biomarkers in a microplate using an antibody specific for each of the biomarkers.

11. The method according to any one of the preceding claims, wherein the subject is a human subject.

12. *In vitro* use of a kit comprising reagents for measuring the expression levels of a panel of biomarkers in a biological sample from a subject according to the method of any of the preceding claims.
